# EUROPEAN PATENT APPLICATION

(11) **EP 0 872 549 A1**
(43) Date of publication of application: **21.10.1998**
(21) Application number: 96927965.2
(22) Date of filing: 21.08.1996
(51) Int. Cl.: C12N 15/00, A61N 2/00, A61N 5/06

(54) **DEVICE "BIOTRON TSZYAN-2" FOR TRANSMITTING A NATURAL INFORMATION SUPPLY TO A BIOLOGICAL OBJECT**

(30) Priority: 01.09.1995 RU 95114538
(71) Applicant: Tszyan Kanchzhen, Jury Vladimirovich, Khabarovsk, 680000 (RU)
(72) Inventor: Tszyan Kanchzhen, Jury Vladimirovich, Khabarovsk, 680000 (RU)
(74) Representative: Sparing - Röhl - Henseler Patentanwälte
(86) International application number: RU9600236
(87) International publication number: WO9641872

(57) **Abstract**

The proposed device comprises a chamber assembly with a main housing (1) and two antenna systems mounted on opposite sides of the housing (1) and each provided with a reflector (6,8) and microwave lens mounted coaxially with the respective reflector. The device is provided with means for housing a biofield source (3) and for housing a biological object (5), located in compartments (2, 4) formed by part of the main housing (1) and antenna system mounted thereon. Young plants or animals can be used as the biofield source (3).

## Description

### Field of the invention

The present invention relates to the field that ensures the maintaining of vitality of a biological object or possible change of its features, namely, to a device for transmitting a natural information supply to a biological object. This device allows to transmit to the object a genetical information which is stored in deoxynucleic acid (DNA) of the source that is located at a distance from the object by means of the influence of a bioelectromagnetic field to the object's molecules.

### Background of the invention

A method of transmitting genetical material to the cells of Actinomyces is known in the art.

Also known is generation of new species of animals by means of separation of a gene coding a specific hormone homologous to that available at the ovum. (EP, Application # 0061253, Cl. 12N 15/00, 1982).

However, in these cases it is necessary to interfere into the cell's structure which involves some technical difficulties; there is a necessity of observance of the sterility of the experiment, and the use of precision equipment. (PCT, Application WO 088(08026, Cl. C12N 15/00, 1988).

Besides, these methods do not ensure the possibilities of rejuvenescent and curing effects on the biological object, especially a human being. To continue vital activity a live body constantly receives from its environment an energy supply coming from air and foods which contain proteins, fats, carbohydrates, vitamins, mineral substances, and water. They contribute to its growth and metabolism. However, they can not slow down the process of aging.

In the process of its vital activity the body's atoms and molecules are interconnected by bioelectromagnetic fields that are unitary material carriers of biological information and energy. Replenishment of the biofield of the object or its individual organs can be effected by transmitting a natural information supply received from another live object.

There is known a device for transmitting a natural information supply to a biological object comprising a source of biofield and a means for housing the source and the object (SU, Inventor's Certificate # 1593670, Cl. A61N 5/06, 1989).

As a source of biofield can be used the hands of the operator who is carrying out specific movements of his hands near the body of the object. Simultaneously with this, the biological object is influenced by an alternating electric field and infrared radiation. The operator is a source of superhigh frequency radiation. And due to the fact that any live cell is a small radiator of electromagnetic waves of a superhigh frequency band, the operator transmits to the object/receiver a biological information, replenishes the biofield of the object/receiver and energizes it. In doing so, the curing effect takes place.

However, in this case is used a source of information supply of the same species as the biological object/receiver. In doing so, the source is used many times for working with various objects. But it is known that biofield exerts mutual influence. That is why, the source receives from the object a pernicious influence that can be transmitted to other objects during his intercourse with them. Besides, the influence of the biofield is not so efficient because it takes place directly between two biological objects and is not amplified by any instruments.

### Summary of the Invention

This invention is aimed at the solution of a technical task of elimination of a possible pernicious influence on a biological object of a source's biofield that has been harmfully affected during its intercourse with another object; the invention is also aimed at the raising of efficiency of the beneficial influence on the object and its consumption of a natural information supply.

The raised task of the invention is attained due to the fact that the device for transmitting a natural information supply to a biological object includes a source of biofield and means for housing the source and the object and comprises a chamber assembly having a housing, two antenna systems each of which having a reflector and a microwave lens mounted coaxially with the respective reflector, the first antenna system being secured to one side of the housing in such a manner as to form a compartment for reception of an information supply from the source of biofield while the second antenna system-is secured to the opposite side of the housing to form a compartment for influencing a biological object, means for placing a source of biofield and the biological object being located in the zone of focus of the respective antenna systems while near the latter means from the side opposite to the antenna system is mounted a group of microwave lenses.

Implementation of the said device in the form of a chamber assembly, comprising a housing and two antenna systems as well as availability of an additional system of lenses and its location at a focus distance from a biological object, ensures the possibility of a more complete taking away of an information supply and its focussed transmittance to the object. Implementation of said antenna systems in the form of a reflector and location of a microwave lens mounted coaxially with it (or secured to it) ensures the increase of the amplification coefficient and the directional action of the systems.

Said housing may have a cylindrical form with antenna systems being secured to its end sides. Implementation of said housing in this form ensures easiness of use of the device which is manifested in the possibility of easy replacement of the source of biofield and, as a result, in shortening of time needed for carrying out this operation.

Said housing may have such a, design that together with said antenna systems secured to its opposite sides there is formed a chamber in the form of a sphere or a chamber the section of which has the form of an ellipse. Such a form of the chamber ensures high mechanical rigidity and high percentage of a natural information supply received by the object.

It is recommended to separate said compartments by a partition secured inside said housing and made of such a material that the bioelectromagnetic radiation can penetrate through it. This creates more comfortable conditions in the compartment for a bioobject due to elimination of the possibility for the smell and noise from the source of a biofield to penetrate into it.

In case of the necessity to transmit a natural information supply to a small object, for example, embryos, cells, it is necessary to supplement the second antenna system with a convex metal mirror located in the zone of the focus of the reflector and lens and facing with its convex side the reflector thus ensuring concentration of electromagnetic radiation of the biofield into a narrow beam for its direction onto a small object. In this case the total resulting focus of said antenna system will shift closer to said reflector. A small biological object placed into that zone will receive an electromagnetic flow of higher density resulting in the increase of its effect.

It is recommended to use as a source of biofield some young plants with the period of vegetation of 1 to 2 weeks from the beginning of vegetation or some large or small animals at the age up to the first half of their growth. At such a stage of growth cells are in an active state of division and, as a result, have more active biofields due to which the effect on the biological object/receiver is greater.

Such a design of said device allows to eliminate a pernicious effect of one biological object-receiver on the other which may take place when the influence on objects/receivers is exerted only by one operator used as a source of biofield. With the present device in each case is used an individual, young, healthy, and strong source of biofield which has not yet interacted with another biological object. Besides, during the course of treatment the source of biofield may be easily changed to a new one several times. This results in the raising of the degree of transmittance of a full-fledged natural information supply and, consequently, a fuller replenishment of the biofield of the object which gives positive results in treatment, rejuvenation of human being or change of features of an animal or a plant.

### Brief Description of the Drawings

This invention will be apparent from the detailed description of the preferred embodiment of the invention with reference to the accompanying drawings in which:
FIG.1 shows the device for transmitting a natural information supply to a biological object having a cylindrical housing.
FIG.2 is a section along A-A of FIG. 1.
FIG.3 is a section along B-B of FIG. 1.
FIG.4 is a section along C-C of FIG. 1.
FIG.5 shows a fastening assembly of the antenna system's reflector.
FIG.6 shows the device for transmitting a natural information supply having a chamber of a spherical form.
FIG.7 shows the device for transmitting a natural information supply to a biological object having a chamber of elliptic section.
FIG.8 shows the second antenna system supplemented with an additional mirror.

### The Preferred Embodiment of the Invention

The device for transmitting a natural information supply to a biological object comprises a chamber assembly having housing 1 made, for example, of duralumin and two antenna systems. The first antenna system is a receiver of a bioelectromagnetic radiation and is secured to one side of housing 1 forming, together with an adjacent part of housing 1, compartment 2 for reception of an information supply from source 3 of biofield. The second antenna system serves for reception and directional transmittance of a bioelectromagnetic radiation and is secured to the opposite side of housing 1 forming, together with an adjacent part of housing 1, compartment 4 for influencing biological object 5. The first antenna system comprises reflector 6 and microwave lens 7 mounted coaxially with it. The second antenna system comprises reflector 8 and microwave lens 9 mounted coaxially with it.

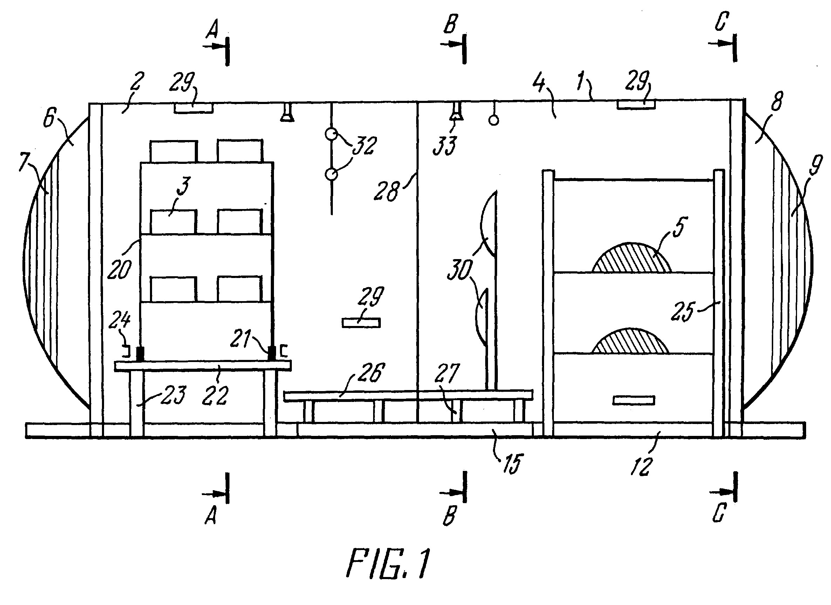

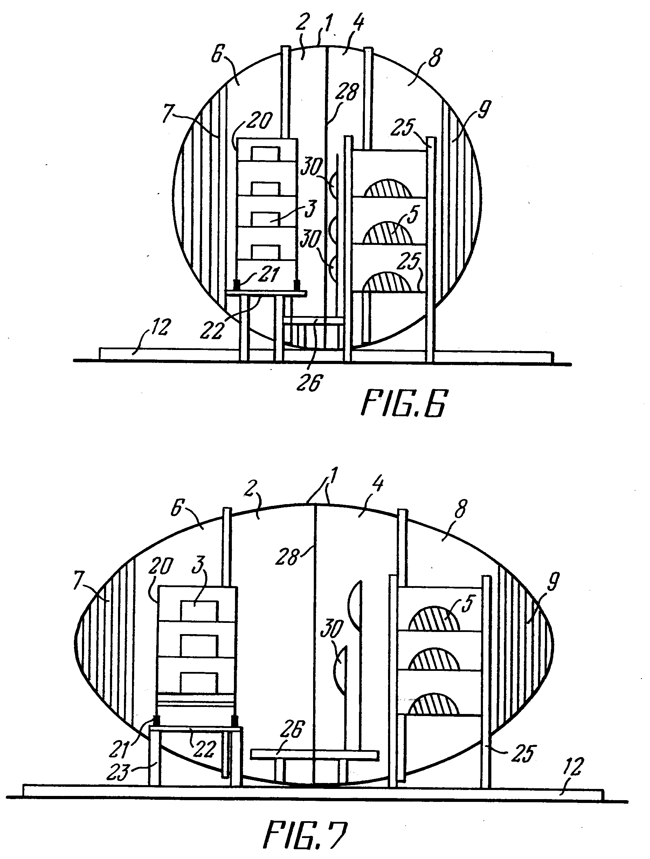

Housing 1 may have a cylindrical form (FIG.1). Said antenna systems should be secured to its end sides. In doing so, it is recommended to use reflectors 6,8 having a parabolic form. Housing 1 may have such a design that together with said antenna systems, secured to its opposite sides, it forms a spherical chamber (FIG.6).

Housing 1 may have such a design that together with said antenna systems secured to its opposite sides it forms an elliptic chamber (FIG.7).

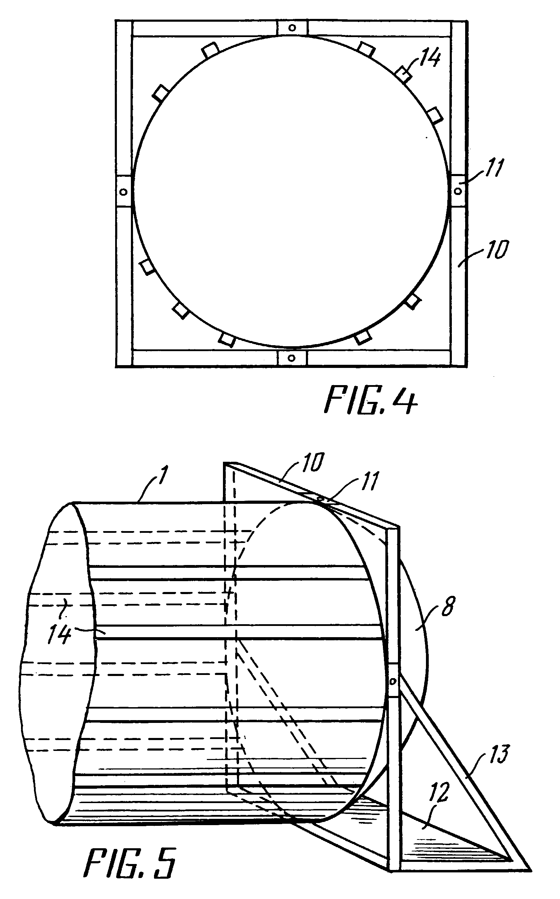

Each reflector 6,8 is secured by its end side to mounting frame 10 (FIG. 4, 5) by means of eyes 11. Said frame has a support system comprising plate 12, mounted on a foundation, and inclined columns 13. Each reflector 6, 8 at its edges has flanges to which are secured rods 14 which encompass cylindrical housing 1 to ensure its rigidity. Housing 1 has a support made in the form of bed 15.

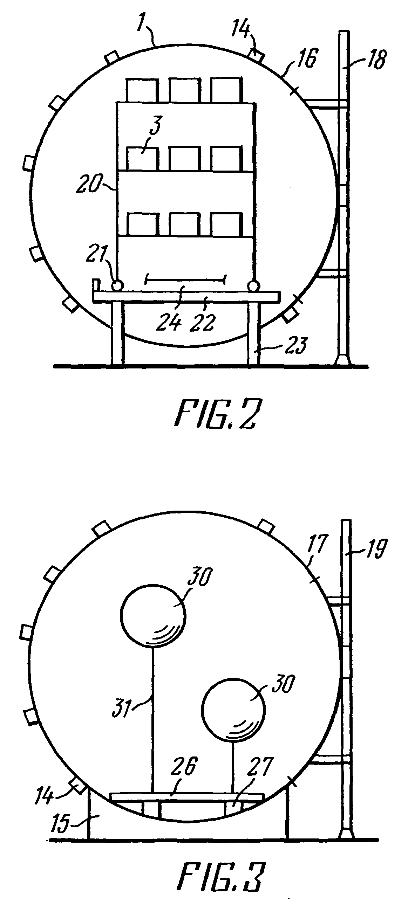

In housing 1 for compartments 2 and 4 are provided doors 16, 17 (FIG. 2, 3) being a part of the surface of housing 1 and having for the cylindrical design of housing 1 a form of an arc. In spherical or elliptic designs of said chambers doors 16, 17 have the forms of a part of a sphere and a part of an ellipsoid respectively.

Doors 16 and 17 are secured to columns 18, 19 by means of hinged joints (FIG. 2, 3).

In compartment 2 in the zone of focus of the first antenna system is provided means for placing source 3 of biofield; such means may be designed in the form of movable shelf 20 (FIG.1, 2, 6, 7) having wheels 21. Shelf 20 is mounted on support platform 22 located opposite door 16. Platform 22 is secured on columns 23 passing through slots in housing 1 and is supported on said foundation. Platform 22 has side guides 24 (FIG.1, 2) for wheels 21 of shelf 20. Behind this assembly is installed a limiter of shelf movement (not illustrated).

In compartment 4 in the zone of focus of the second antenna system is provided a means for placing of one or more biological objects 5; said means is made in the form of multitier or single-tier bed 25 (FIG.1,6,7) supporting elements of which through said slots in housing 1 are mounted on said foundation.

Housing 1 has floor 26 for people to move around. Supports 27 for said floor are located in such a way that they through cylindrical housing 1 are supported on bed 15.

Compartments 2 and 4 may be separated by means of partition 28 (FIG.1) secured in housing 1 and made of the material that can be penetrated by the bioelectromagnetic field, for example, of polyethylene (or coloured polyethylene) or foam plastic.

Housing 1 has several apertures 29 (FIG. 1) covered with a small mesh brass lattice (mesh size: up to 1 mm) for communication with the environment.

Near said means for placing a biological object at the side opposite to the second antenna system is mounted a group of microwave lenses 30 (FIG.1, 6, 7) the number of which corresponds to the number of tiers of bed 25 used for placing bioobjects. Support 31 of lens system 30 is located on floor 26. It is possible to provide a regulated movement of said lenses horizontally and vertically. Total area of all said lenses must not exceed 10% of the cross-sectional area of housing 1.

In housing 1 are installed conventional lamps 32 and quartz lamps 33.

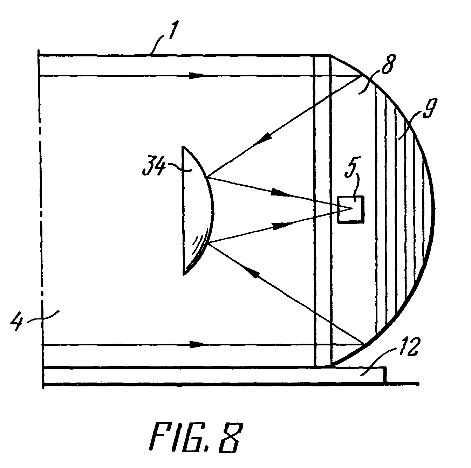

The second antenna system may be supplemented additionally with convex metal mirror 34 (FIG.8) located in the zone of reflector 8 and lens 9 and facing by its convex side said reflector to ensure the concentration of the electromagnetic radiation of biofield into a narrow beam. It is recommended to use it in case of influence on a small biological object.

The operation of said device for transmitting a natural information supply to a biological object shall be performed in the following way.

Through door 17 biological objects, for example, several people, enter conpartment 4 and are placed on tiers of bed 25 while through door 16 to compartment 2 is delivered movable shelf 20 on the shelves of which are placed sources 3 of biofields. In the capacity of such sources may be used young plants with the period of 1 to 2 weeks from the beginning of vegetation, for example, wheat, corn, peas, soy beans, cabbage, flowers grown in flower pots and their crops without thorns. Seeding of grains shall be done so densely that adjacent grains must contact each other. During the course of treatment the pots with the plants must be replaced every 2 hours. As a source of biofield may also be used animals at the age of half the period of their growth (for example, mice, hares, rabbits, dogs, deer, bears, etc). They shall be put into cages made of non-metallic material. The duration of a course of treatment must be from 2 to 4 hours daily for replenishment of the biofield and 8 hours daily for rejuvenation. It is recommended to proceed 10 courses of treatment.

The use of biofields of animals is allowed only for those persons who do not plan to have children further on.

The first antenna system accepts the bioelectromagnetic radiation from that side of source 3 of biofield that faces that system, forms and opens a flat phase front and directs it to second antenna system 4. Then, from that system said bioelectromagnetic radiation is focused into the focus zone of that system where is located biological object 5 (or several objects 5). In that zone is formed a structure of said bioelectromagnetic field similar to that of the source. That biofield exerts influence on object 5 ensuring transmittance to it of a natural information supply.

The bioelectromagnetic radiation from the side of source 3 of biofield that does not face the first antenna system is received by first microwave lens group 30, is focused and transmitted by those lenses to specific areas of biological object 5 that especially are in need of reception of a natural information supply.

The device can also be used for transmitting a natural information supply not only to a human being but also to bioobjects of other species. For example, one can place into compartment 4 in the zone of focus of the second antenna system germinated seeds of vegetable and grain crops, fruits, fodder grass, medicinal plants, flowers, tree seeds, etc. As source 3 of biofield shall be selected such species the features of which is necessary to transmit to the object/recipient. For example, if the objective is to grow corn with multiple stems, high yield and grains containing higher amounts of proteins. In this case as source 3 of biofield must be used wheat crop. The result achieved: crop yield is from 30 to 100% higher than in the reference group, with a higher average content of protein.

Or if, for example, it is planned to grow cucumbers having a specific scent and taste. For that purpose as source 3 of biofield must be used green mass of melon (stem, leaves) or fruits of pine-apple, apples, mandarins, etc. The resulting cucumbers have a taste of melon, pine-apple, etc., i.e. the taste of the biofield source. The duration of the treatment course is 3 to 4 days without interruption but source 3 shall be changed to a new one approximately every 4 hours. Germinated seeds during the course of treatment shall be maintained moistened, and are washed 2 or 3 times a day with pure water. After the treatment seeding in the field may be implemented.

It is also possible to exert such influence on embryos, cells, and tissues of animals. In doing so, it is expedient to use the device (FIG.8), in which the second antenna system is provided with convex metal mirror 34 located in the zone of focus of reflector 8 and lens 9. Biological object 5 is located in the zone of focus of the whole antenna system with that focus being shifted closer to reflector 8. The bioelectromagnetic radiation directed from the first antenna system comes to reflector 8 and lens 9 and from them - to convex mirror 34. From that point the electromagnetic flow is reflected and in the form of a more dense beam is directed to the zone of focus of the second antenna system where biological object 5 is located. Thanks to higher concentration the degree of influence during transmitting to an object a natural information supply is increased.

### Industrial Applicability

The device is easy to manufacture and use, it is recommended to use it for maintaining vitality of bodies by way of transmitting to them a natural information supply from live sources.

## Claims

1. The device for transmitting a natural information supply to a biological object comprising a source of biofield and means for housing said source and object characterized in that said device comprises a chamber assembly having a housing (1) and two antenna systems each of which has a reflector (6, 8) and a microwave lens (7, 9) mounted coaxially with it, the first antenna system being secured to one side of said housing (1) forming compartment (2) for reception of an information supply from a source (3) of biofield while the second antenna system is secured to the opposite side of said housing (1) forming a compartment (4) for exerting influence on a biological object, the means for housing a biofield source and a biological object are located in the zone of focuses of the respective antenna systems, and near the last means from the side opposite to the antenna system is mounted a group of microwave lenses (30).

2. The device according to claim 1, characterized in that a housing (1) has a cylindrical form and the antenna systems are secured to its end sides.

3. The device according to claim 1, characterized in that a housing (1) is designed in such a way as to form, together with the antenna systems secured to its opposite sides, a chamber having a spherical form.

4. The device according to claim 1, characterized in that a housing (1) is designed in such a way as to form, together with the antenna systems secured to its opposite sides, a chamber the section of which has a form of an ellipse.

5. The device according to claim 1, characterized in that compartments (2, 4) are separated by a partition (28) secured in a housing (1) and made of such a material that can be penetrated by the bioelectromagnetic field.

6. The device according to claim 1, characterized in that the second antenna system is supplemented with a convex metal mirror (34) located in the zone of focus of a reflector (8) and lens (9) and facing by its convex side said reflector (8) ensuring concentration of the electromagnetic radiation of biofield into a narrow beam for its direction onto a small biological object (5).

7. The device according to claim 1, characterized in that as a source (3) of biofield are used young plants with the period from 1 to 2 weeks from the beginning of vegetation.

8. The device according to claim 1, characterized in that as a source (3) of biofield are used large or small animals at the age up to the half of the period of their growth.
